# EUROPEAN PATENT APPLICATION

(11) **EP 2 417 971 A1**
(43) Date of publication of application: **15.02.2012**
(21) Application number: 10171213.1
(22) Date of filing: 29.07.2010
(51) Int. Cl.: A61K 31/138, A61P 15/08

(54) **Clomiphene for use in increasing the chances of pregnancy of women having a low ovarian reserve**

(71) Applicant: Nitzschke, Markus, 53127 Bonn (DE)
(72) Inventor: The designation of the inventor has not yet been filed
(74) Representative: HOFFMANN EITLE

(57) **Abstract**

The present invention provides compounds for use in a method for improving the chances of a female with a low ovarian reserve to become pregnant. This is achieved by the intake of Clomiphene or a pharmaceutically acceptable isomer, salt or hydrate thereof on the 6^{th} or 7^{th} day of the menstrual cycle and is continued until the maturation of the oocyte. The intake of Clomiphene results in the inhibition of a pathological premature LH-rise, which occurs in women with a low ovarian reserve. This treatment allows an immature oocyte to mature to a fertilizable oocyte which can subsequently be fertilized either by IVF or in a natural way.

## Description

### Field of the Invention

The present invention provides compounds for use in a novel treatment method for maturation of otherwise non-fertilizable oocytes in a female with a low ovarian reserve. This method serves to increase the chances of such women to become pregnant, both naturally and by way of in vitro fertilization (IVF).

### Background of the Invention

Nowadays the percentage of women being already in an age of 35 or older when coming to the decision to have a baby has risen significantly due to changed personal circumstances, family structures, position of women in our society, etc. Quite often this group of women can no longer become pregnant in the natural way. This is mostly due to a higher percentage of women with a low ovarian reserve in this age group. Triggered by this development the reproduction medicine has made significant progress in treating women with various pathologies.

The number of oocytes in a female human gradually decreases with age. When the female is approaching the menopausal stage, the number of oocytes is low and gradually even decreases to zero in the menopause. This state of pre-menopausal women is denoted as "low ovarian reserve" (also known as reduced ovarian reserve, compromised ovarian reserve, poor ovarian reserve, impaired ovarian reserve or declining ovarian reserve) and generally means that the chances of pregnancy are drastically impaired.

The following is considered to reflect the current consensus opinion about the treatment options of women with low ovarian reserve (citation from the website http://www.ivf.com/ovarianreserve.html):
"When further testing confirms compromised ovarian reserve, it is important to point out that the likelihood of success with infertility treatment is low. Such patients may want to consider other options, such as donor-egg treatment. However, we realize that donor eggs will not be acceptable to everyone with poor ovarian reserve.
   We have found that when ovarian reserve is low, the best course of action for the physician is compassionate honesty. After all, time is precious by the time a couple reaches infertility specialists, so little can be gained by creating false hope or continuing down a fruitless path

Besides the fact that a state of low ovarian reserve implies a low number of (theoretically) fertilizable oocytes to begin with, certain disorders in the ovulation process, which prevent a desired pregnancy may occur in this state. One of these disorders/pathologies that has not yet been described in the literature is characterized by a premature luteinizing hormone (LH) surge when the follicle is still small and unripe. A healthy oocyte normally matures in a growing follicle within a period of 10 to 14 days, until the follicle has reached a size of about 18 to 20 mm. Starting from a certain value, which is usually reached at the middle of a menstrual cycle, the estrogen which is produced by the follicle, triggers a LH-surge and hence the ovulation process.

In women with a reduced ovarian reserve, a specific inhibition or down regulation process of LH during oocyte maturation appears to be lacking, since the LH-surge often occurs before the full maturity of the follicle. As a consequence, oocytes which are obtained from such immature follicles are often of low quality and cannot be fertilized in most of the cases.

In the past, various attempts have been made to help pre-menopausal women who were unable to become pregnant. The most common treatment for infertility in general, is in-vitro fertilization (IVF).

For 25 years, the so-called "long gonadotropin releasing hormone agonist (GnRHa) pituitary suppression regimen" with relatively high doses of exogenous follicle combined-stimulating hormone (FSH) remained the most frequently used stimulation protocol for IVF. The success of this protocol is mostly based on the fact that pituitary suppression and exogenous ovarian stimulation can be easily learned and reproduced. However, patients with low ovarian reserve and high basal FSH are often unable to respond sufficiently to ovarian stimulation and their treatment cycles are often cancelled. Patients with a low ovarian reserve also seem to be more sensitive to high doses of FSH in terms of oocyte quality. Even if a sufficient number of oocytes can be retrieved after FSH stimulation in these patients, fertilisation and implantation rarely occurs.

Yet another stimulation protocol for IVF, which has recently been described by Teramoto et al. Reproductive BioMedicine Online, Vol. 15, No. 2, 2007, p. 134-148, involves the use of 50 mg Clomiphene citrate from cycle day 3 onwards. This new protocol is designated as "minimal ovarian stimulation". It is not designed for women with low ovarian reserve in particular. Clomiphene is administered in this method for a relatively long period of time, i.e. 10-12 days until the day before maturation is triggered by administration of a GnRH agonist. Oocytes are then retrieved 32-35 h later. By this method Teramoto et al make use of the antagonistic action of Clomiphene citrate to the estradiol receptor on the hypothalamus level, inhibiting both positive and negative feedback, and resulting in the induction of the ovarian stimulation and suppression of ovulation.

As Teramoto et al (supra) noted, the mechanism of Clomiphene is still "shrouded in mystery", even though its anti-estrogenic action and its action to prevent the premature LH surge, which represents the biggest problem in IVF, were described and used in his protocol. The term "premature LH-surge" as used by Teramoto and other authors means a LH-surge naturally produced in the body during a stimulation of the ovaries in preparation for an in-vitro fertilization (IVF). If the LH rises, triggered by the own body functions, the ovulation cannot be induced in a controlled manner anymore and one is forced to retrieve the ovum 36 h after the start of the LH-surge.

Teramoto has therefore used Clomiphene, inter alia, to control the ovulation process in the course of an in vitro fertilization (IVF). To make use of both the stimulating and the inhibiting effect of Clomiphene, Teramoto et al had to administer Clomiphene right at the beginning of the menstrual cycle until the day of induction of ovulation, which is usually between the 10^{th} and 12^{th} day of said cycle. The stimulating effect occurs prior to the 6^{th} cycle day, before a dominant follicle is selected, whereas the inhibiting effect starts on day six or seven after selection of the dominant follicle. However, as we observed, if Clomiphene is administered in the described manner, the growth of the endometrium is inhibited at the same time due to the anti-estrogenic effect of Clomiphene (blocking of the estrogen receptors) and thus the embryos should not be transferred in the same cycle, because implantation success rate is low. In order to increase the implantation success rate, the resulting embryos should be frozen and transferred in the next cycle where no Clomiphene medication is conducted.

The object of the present invention is to provide an alternative mild treatment of women having a low ovarian reserve, which allows such women to produce oocytes of high quality that can be fertilized naturally, inside the woman's body, or by in-vitro fertilization (IVF).

### Summary of the Invention

The inventors have found that the maturation of oocytes in such women having low ovarian reserve can surprisingly be promoted and hence chances of pregnancy be increased by the administration of (preferably) low doses of Clomiphene or an isomer, salt or hydrate thereof from day 6-7 of the menses (or once selection of a dominant follicle has occurred and this follicle reached a size of 12-14 mm) onwards up to the maturation of the oocyte.

According to a preferred embodiment of the present invention, the maturation of the oocyte to a fertilizable ovum is promoted by the administration of low doses (25 mg per day or 50 mg every 2^{nd} day) of Clomiphene from day 6-7 of the menstrual cycle (menses) or when the follicle has reached a size of 12-14 mm, onwards up to the maturation of the oocyte.

Without wishing to be bound by theory, it is assumed that the described promotion of the maturation occurs through an inhibition of a pathological premature luteinizing hormone (LH) rise, which otherwise frequently occurs in females with a low ovarian reserve. Compounds that were found useful in such a method are Clomiphene and its pharmaceutically acceptable isomers, salts and hydrates, when administered according to a certain administration regime. In a particularly preferred embodiment, Clomiphene citrate is used. The invention is further directed to the use of Clomiphene to obtain a fertilizable ovum from a female with a low ovarian reserve.

In the context of the present invention, the term "pathological premature LH-rise" describes the situation, where the LH rises at a stage where the dominant follicle is small and immature and estrogen levels are still low. This pathological situation, which has not been described in the literature before, frequently occurs in women with a reduced ovarian reserve.

### Detailed Description of the Invention

As mentioned before, a woman is born with a certain amount of oocytes in the ovaries. With the advanced age the number of oocytes that can be successfully recruited for a possible pregnancy declines. At the stage of the menopause the oocytes are completely depleted.

One distinguishes between different states of ovarian insufficiency: The first grade is an elevated level of the follicle stimulation hormone (FSH) at the beginning of each cycle. As long as the menstrual cycle is still regular and longer than 26 days, the low ovarian reserve has only little clinical relevance. In general, these women are known as low responders. To achieve a multiple follicular growth, it is common practice to use a higher dosage of FSH for stimulation. The problem is, however, that a high FSH level during follicular growth results in a down regulation of the FSH receptor at the follicles. Moreover, high FSH levels during follicular growth seem to affect the ovum quality.

In the second stage of ovarian insufficiency, the menstrual cycle gets shorter than 26 days. The pituitary suppression during the luteal phase is not strong enough. A higher FSH baseline level stimulates the follicular growth already in the middle of the luteal phase, which leads to a shorter follicular phase. At the onset of menstrual bleeding, the woman has already developed a dominant follicle, which will ovulate too early. The result is a desynchronisation of follicular growth and endometrial maturity.

In the third stage of ovarian insufficiency, which is in the focus of this invention, women also have a shorter menstrual cycle. However, the shorter cycle is not caused by a simple desynchronisation of the follicular growth and endometrium maturity, but according to our observations most probably by an early pathological luteinizing hormone (LH)-rise at a stage where the dominant follicle is still immature and the estrogen level is still low. The cause of this pathological premature LH rise is still unknown, but its consequences are quite severe: If one tries to retrieve an oocyte from these small follicles, this results in an immature ovum or poor embryo quality. Since the baselines of FSH and LH are higher in these patients, the positive feedback of estrogen during follicular growth seems to have a stronger effect on the hypothalamus.

The present invention is therefore directed to the use of Clomiphene (including Enclomiphene) and their pharmacologically acceptable salts in a method for improving oocyte maturation and increasing the chances of pregnancy in a female with low ovarian reserve who profits from an inhibition of the pathological early LH rise. It has surprisingly been found that the ovary-stimulating effect of Clomiphene, which has formed the only or at least the dominant rationale for the administration of this drug for decades, is neither necessary nor even desirable to help this type of women to a pregnancy. The administration of Clomiphene is therefore only commenced on day 6 or 7 of the menstrual cycle, when selection of a dominant follicle has already occurred, contrary to the established rule to use Clomiphene directly from the beginning of the menstrual cycle. It is terminated when the oocyte and the follicle containing it are mature, i.e. shortly before ovulation naturally occurs or is triggered.

In another aspect, the present invention pertains to a method for increasing the chances of a woman having a low ovarian reserve to become pregnant, wherein the method comprises the administration of an effective amount of Clomiphene from day 6 or 7 of the menses onwards until the maturation of the oocyte.

Clomiphene, as it is on the market, is a mixture of two geometric isomers, namely Enclomiphene (E-Clomiphene, transisomer) and Zuclomiphene (Z-Clomiphene, cis-isomer). Enclomiphene has a shorter half-life time in the body than its isomer Zuclomiphene. According to the invention, Clomiphene is preferably used in the form of one of its pharmaceutically acceptable acid addition salts or hydrates, and Clomiphene citrate is the most preferred embodiment. Alternatively, the isomer Enclomiphene may be used in isolation, preferably in the form of the citrate or hydrochloride. The term Clomiphene, whenever used in this application, is to particularly include Enclomiphene and its pharmaceutically acceptable salts, either as a pure isomer or as part of the isomer mixture in Clomiphene.

According to the present invention and in contrast to the common use of Clomiphene in the prior art as described above, Clomiphene (or an isomer thereof or a salt of either of them) is only administered on the 6^{th} or 7^{th} day of the menstruation cycle, when a dominant follicle has been selected, not before. At this stage, the growth of the endometrium is already completed. Hence, despite the intake of Clomiphene, the embryos retrieved in this cycle can directly be transferred in the same cycle without freezing them in the meantime. The Clomiphene treatment is continued until the follicle is mature and ovulation occurs or is triggered. In view of the half-life of Clomiphene and its isomers, it is possible to give the last dose of the drug one day before the ovulation is triggered or occurs. If the dose is higher, e.g. 50 mg orally, it is even possible to give the last dose two days before ovulation induction. Each of these cases is included in the term "until the maturation of the oocyte"

At this point the ovulation can be triggered. After the ovulation a pregnancy can either be achieved in the natural way, inside the body of the woman, or by IVF.

In order to obtain the desired pharmacological effects according to this invention, Clomiphene can be administered in only very low doses. Suitable doses are 10 - 35 mg / day, preferably 25 mg/day, or 40 - 60 mg, preferably 50 mg every 2^{nd} day until the oocyte within the follicle is mature. The exact dosage should be determined by the responsible physician on the basis of the desired inhibitory effect on the pathological premature LH rise and potential side effects of Clomiphene.

The administration can be orally, which is preferred, but also by other convenient means, i.e. by way of an infusion, or transdermally by way of a patch. The same applies to the individual isomers of Clomiphene, particularly Enclomiphene, and their pharmacologically acceptable salts (the mg amount is to be understood as the amount of the active ingredient in its free form, i.e. without the salt anion).

### Examples

### General procedure

Women with low ovarian reserve take 50 mg oral Clomiphene Citrate every second day or 25 mg each day after the 6^{th} day of the menstrual cycle until the follicle has reached a desired size of 18-20 mm. Subsequently ovulation is artificially triggered by the administration of GnRH (Gonadotropin releasing hormone) analogs, which is preferred, or human chorionic gonadotropin (HCG), 36-40 h after the administration of the ovulation inducing agent, the women had sexual intercourse. Alternatively, the ovum was obtained after 36 h and was fertilized in vitro in the lab.

### Case Study

A 41-year-old nulliparous woman with 6 years of infertility had regular short cycles of 23 days. When she was younger, she had longer cycles of up to 29 days, but starting at the age of 34 years, her cycles became gradually shorter. She had a normal baseline FSH (6.3 IU/mL) at day 3 of her cycle, but her serum estradiol (110 pg/mL) was too high for this day. When a vaginal ultrasound scan was performed that day, a follicle of 14 mm on the left ovary was found. The woman had a normal hysterosalpingogram, and no pathology could be found at laparoscopy or hysteroscopy. Her husband's semen analysis was normal. They had failed six cycles of gonadotrophins-IUI (intra-uterine inseminations) and 4 IVF attempts. The last two cycles had to be cancelled due to low response in a GnRH antagonist protocol with FSH injections of up to 450 IU per day. On day 7 of her cycle a hormonal assay and vaginal ultrasound scan were performed. She had a follicle of 17 mm on her left ovary, serum estradiol of 213 pg/mL and LH 32 IU/L. Her endometrium lining had only 5.8 mm and her menstrual bleeding stopped only two days ago. The women came back two days later. The follicle was gone and an oral contraceptive pill (OCP) for 10 days was started in order to prevent follicle growth during luteal phase. Five days after the last OCP, menstrual bleeding started. No dominant follicle could be seen, serum estradiol was 32 pg/mL and FSH 14 IU/L.

The woman was again examined on day 9 of her cycle. She had a dominant follicle of 15mm on the right ovary, serum estradiol 134 pg/mL and LH 22. Since oocytes ovulated from follicles smaller than 16 mm are often of a poor quality and do seldom produce a pregnancy, it was refrained from trying to retrieve the egg this time. The woman ovulated the next day.

The woman was examined again on day 6 of her next cycle. She had 2 small follicles of 12mm and 11mm on her left ovary. Serum estradiol was 113 pg/mL, FSH 7.7 IU/L and LH 5.6 IU/L. In order to avoid another pathological premature LH rise on a small follicle this cycle, the woman was treated with small doses of Clomiphene citrate, i.e. 50mg Clomiphene citrate on day 6, 8 and 10 of the cycle. On day 11 two follicles on the left ovarian was found with sizes of 19mm and 15mm. Serum estradiol was 621 pg/mL and LH 7.4 IU/mL. Ovulation induction with 0.2mg triptorelix was performed on the evening of the next day and an oocyte was retrieved 36 hours later. Two mature eggs could be retrieved, but only one oocyte could be fertilized by IVF. Vaginal progesterone treatment was started the day of the egg retrieval. The embryo was transferred at day 2. Two weeks later the pregnancy test was positive.

## Claims

1. Clomiphene or a pharmaceutically acceptable isomer, salt or hydrate thereof for use in a method for improving the chances of a female with a low ovarian reserve to become pregnant, wherein the administration of Clomiphene or a pharmaceutically acceptable isomer, salt or hydrate thereof is started on the 6^{th} or 7^{th} day of the menstrual cycle, or when the follicle has reached a size of 12-14 mm, and is continued up to the maturation of the oocyte.

2. Clomiphene or a pharmaceutically acceptable isomer, salt or hydrate thereof for use in a method according to claim 1, wherein the method improves the maturation of an oocyte, thus resulting in the development of a fertilizable ovum in a female with a low ovarian reserve.

3. Clomiphene or a pharmaceutically acceptable isomer, salt or hydrate thereof for use in a method according to any of the preceding claims, wherein the female having a low ovarian reserve has not been pretreated in the same cycle with GnRH agonists or GnRH antagonists to down-regulate the pituitary gland or with Clomiphene to stimulate the ovaries.

4. Clomiphene or a pharmaceutically acceptable isomer, salt or hydrate thereof for use in a method according to any of the preceding claims, wherein the isomer is Enclomiphene, or a pharmaceutically acceptable salt or hydrate thereof.

5. Clomiphene or a pharmaceutically acceptable isomer, salt or hydrate thereof for use in a method according to any of the preceding claims, wherein the salt is Clomiphene citrate.

6. Clomiphene or a pharmaceutically acceptable isomer, salt or hydrate thereof for use in a method according to any of the preceding claims, wherein said Clomiphene or pharmaceutically acceptable isomer, salt or hydrate thereof is administered at a dose of 10-35 mg per day, or 25-75 mg every second day.

7. Clomiphene or a pharmaceutically acceptable isomer, salt or hydrate thereof for use in a method according to any of the preceding claims, which is administered in a dose of 25 mg per day.

8. Clomiphene or a pharmaceutically acceptable isomer, salt or hydrate thereof for use in a method according to any of the preceding claims, which is administered in a dose of 50 mg every 2^{nd} day.

9. Clomiphene or a pharmaceutically acceptable isomer, salt or hydrate thereof for use in a method according to any of the preceding claims, wherein the thus obtained matured oocyte is retrieved after ovulation and fertilized by IVF without an intermediate freezing step.

10. Clomiphene or a pharmaceutically acceptable isomer, salt or hydrate thereof for use in a method according to any of the preceding claims, wherein the thus obtained matured oocyte is fertilized in the natural way after ovulation.

11. Clomiphene or a pharmaceutically acceptable isomer, salt or hydrate thereof for use in a method according to any of claims 9 or 10, wherein the ovulation is triggered by administration of a suitable GnRH agonist, e.g. triptorelin, or by human chorionic gonadotropin (HCG).

12. A method for increasing the chances of a woman having a low ovarian reserve to become pregnant, wherein the method involves the administration of an effective amount of Clomiphene or a pharmaceutically acceptable isomer, salt or hydrate thereof from day 6 or 7 of the menses onwards or when the follicle has reached a size of 12-14 mm, until the maturation of the oocyte.

13. The method of claim 12 wherein the Clomiphene or a pharmaceutically acceptable isomer, salt or hydrate thereof is selected from the group consisting of Clomiphene, Enclomiphene or a pharmaceutically acceptable salt or hydrate thereof, particularly the citrate.

14. The method of claim 13, wherein the effective amount of Clomiphene or a pharmaceutically acceptable isomer, salt or hydrate thereof is 10-35, preferably 25 mg, per day or 25-75, preferably 50 mg, every second day.

15. Use of Clomiphene or a pharmaceutically acceptable isomer, salt or hydrate thereof in a method as claimed in any of claims 12, 13 and 14 to obtain a fertilizable oocyte from a female with a low ovarian reserve.
